Europäisches Patentamt

European Patent Office

Office européen des brevets

(11) Publication number: **0 268 025 B1**

## (12) EUROPEAN PATENT SPECIFICATION

(45) Date of publication of patent specification: **24.04.91**

(51) Int. Cl.⁵: **G01N 33/49**, //G01N33/50, C12Q1/00

(21) Application number: **87112099.4**

(22) Date of filing: **20.08.87**

(54) Method for analyzing a haemolysed blood sample.

(30) Priority: **20.08.86 JP 192676/86**

(43) Date of publication of application:
**25.05.88 Bulletin 88/21**

(45) Publication of the grant of the patent:
**24.04.91 Bulletin 91/17**

(84) Designated Contracting States:
**DE FR GB**

(56) References cited:
**US-A- 4 263 512**

**CHEMICAL ABSTRACTS, vol. 94, no. 23, 08 June 1981, Columbus, OH (US); H.CZERWEK et al., p. 376, no. 189134b**

**CHEMICAL ABSTRACTS, vol. 103, no. 1, 08 July 1985, Columbus, OH (US); D.J.KARKOSKI, p. 291, no. 3123y**

**PATENT ABSTRACTS OF JAPAN, vol. 9, no. 177 (P-375), 23 July 1985**

**DATABASE BIOPASCAL, reference no. 76274658, 1976; N.J. DEACON et al.: "Effect of haemolysis on plasma and serum immunoglobin estimations"**

**JOURNAL OF ELECTRONIC ENGINEERING, vol. 20, no. 204, 1983, Tokyo (JP); I.SAWAMURA: "Computerizing biochemical analysis", pp. 38-41**

(73) Proprietor: **HITACHI, LTD.**
**6, Kanda Surugadai 4-chome Chiyoda-ku, Tokyo 101(JP)**

(72) Inventor: **Sagusa, Hisayuki**
**1280-4, Tabiko Katsuta-shi(JP)**

(74) Representative: **Dost, Wolfgang, Dr. rer. nat. Dipl.-Chem. et al**
**Patent- u. Rechtsanwälte Bardehle, Pagenberg, Dost, Altenburg Frohwitter & Partner Galileiplatz 1 W-8000 München 80(DE)**

## Description

### BACKGROUND OF THE INVENTION

This invention relates to a method for analyzing a blood sample, and more particularly to an analyzing method suitable for measuring components in a haemolyred blood serum or plasma sample .

Most samples to be subjected to biochemical examinations are blood sera or plasma. When various biochemical components are measured using blood serum or blood plasma as a sample, serious errors are present in the results of measurement of said components in some cases depending on propertiesi of the sample itself. In particular, hemolysis, chyle and icterus are three important factors which may result in errors of measurement. Errors due to hemolysis involve special problems.

When hemolysis is caused by destruction of a part of the red blood cells, a blood serum sample or a blood plasma sample separated from the blood clot by centrifugation is contaminated with hemoglobin, resulting in errors of measured values of the components in a sample to be analyzed. US-A- 4,263,512 suggests determination of interfering chromogens in a blood sample to be analysed at the time of measurement of components, and correction of measured values of the components in accordance with the degree of hemolysis.

In this conventional method for correcting measured values, only error due to the color of hemoglobin released from red blood cells by hemolysis is corrected.

### SUMMARY OF THE INVENTION

The object of this invention is to provide a method of analysis which makes it possible to measure components in a blood serum sample or a blood plasma sample of hemolyzed blood by reducing errors of measured values more correctly than conventional methods.

This invention has been made paying attention to the fact that destruction of red blood cells causes contamination of blood serum or blood plasma with various interfering components in addition to hemoglobin. In this invention, measurement errors due to these contaminating components are corrected at the time of measuring the components.

This invention is characterized by comprising the steps of
- obtaining an uncorrected measured value of a component of a blood serum or plasma sample to be analyzed; and
- correcting said uncorrected measured value by the measured degree of hemolysis for said blood serum or plasma sample on the basis of the previously-established correlation between the degree of hemolysis and the amount of said component to be determined in the blood serum or plasma sample to be analyzed.

Various components are contained in red blood cells, and when red blood cells are destroyed, for example, by pretreatment of whole blood, the components contained are released and become indistinguishable from components inherent in blood serum or blood plasma. In particular, components whose concentration is higher in red blood cells than in blood serum or blood plasma produce a large positive error even when slight hemolysis is caused.

In conventional methods, correction of such a positive error has not been taken into consideration, and therefore measured values for a hemolyzed blood sample have been clinically inapplicable in the case of components in which a positive error is produced by hemolysis, such as lactate dehydrogenase (LDH), glutamate-oxaloacete transaminase (GOT) glutamate-pyruvate transaminase (GPT) and, potassium (K). By the way, the ratio of concentration in red blood cells to concentration in blood serum is 200 for LDH, 80 for GOT, 15 for GTP and 28 for K. Moreover, the frequency of occurance of a sample which has undergone medium or higher degree of hemolysis is as high as about 10%, so that the resulting measured values cannot be used as clinical data. This is a serious problem.

The relationship between the degree of hemolysis and the value of error can be established from measurement results for many actual samples obtained by destroying blood cells separated from blood serum. For example, along the axis of abscissa is plotted the degree of hemolysis and along the axis of ordinate is plotted the concentration of a component, and the destribution of the content of the component in red blood cells is investigated, after which based on the result obtained, the slope and the tolerance (the breadth of the distribution) are determined.

Well-known optical measuring methods can be used for analyzing procedure of the component and measuring procedure of the degree of hemolysis. Data useful for diagnosis can be obtained by applying the relationship between the degree of hemolysis and error to uncorrected measured values of the component.

In preferred embodiments of this invention, the relationship between the degree of hemolysis and the value of error is established in terms of a slope and a tolerance attendant thereon, and values obtained by correcting the uncorrected measured values are expressed as values with their respective tolerances. Furthermore, in preferred embodiments of this invention, the slope and the tolerance are determined for each of groups formed by classification according to health condition, age or sex. In addition, the above-mentioned tolerance is determined so as to include all the measured values for many actual samples.

## BRIEF DESCRIPTION OF THE DRAWINGS

Fig. 1 shows a flow chart for explaining an analyzing procedure in one example of this invention.

Fig. 2 is a graph showing one example of error function based on data on total protein obtained for 100 samples from a group under a group medical examination.

Fig. 3 is a graph showing one example of error function based on data on albumin obtained for 100 samples from a group under a group medical examination.

Fig. 4 is a graph showing one example of error function based on data on LDH obtained for 100 samples from a group under a group medical examination.

Fig. 5 is a graph showing one example of error function based on data on GOT obtained for 100 samples from a group under a group medical examination.

Fig. 6 is a graph showing one example of error function based on data on potassium obtained for 100 samples from a group under a group medical examination.

Fig. 7 is a graph showing one example of error function based on data on GOT obtained for 100 samples from a group of inpatients.

Fig. 8 is a schematic diagram showing an outline of the structure of an automatic clinical analyzer to which this invention has been applied.

Fig. 9 is a chart showing one example of input of measurement conditions to the analyzer shown in Fig. 8.

## DETAILED DESCRIPTION OF THE PREFERRED EMBODIMENT

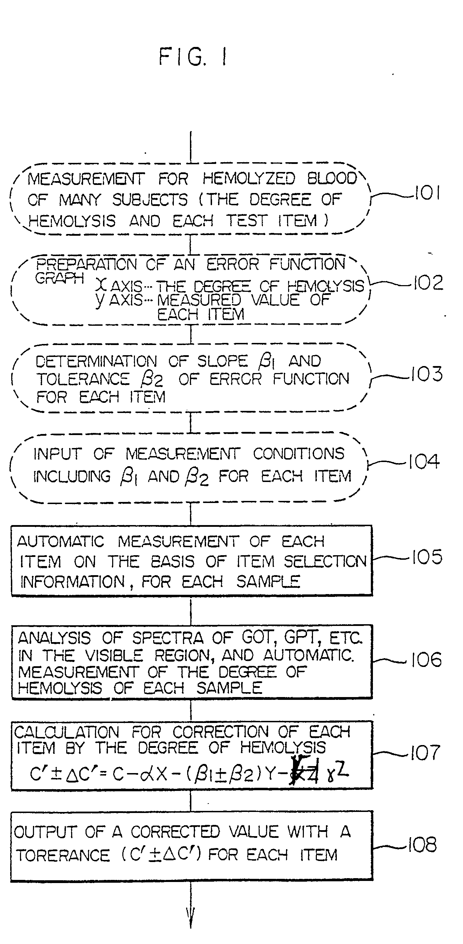

The outline of one example according to this invention is explained below with reference to Fig. 1. Fig. 1 is a diagram for explaining a procedure of correcting measured values of components for the influence thereon of hemolysis: the steps 101 to 104 shown by the dotted lines are preparation steps, and the steps 105 to 108 shown by the solid lines are those of carrying out a routine examination. The steps 105 to 108 are conducted as a part of a conventional biochemical analyzing procedure.

In the step 101, the relationship between the degree of hemolysis and an error produced for each component by hemolysis is established. Blood was collected from each of 100 healthy persons under a medical examination, and blood plasma was separated by means of a centrifuge, after which the blood cell layer was taken out, washed three times with physiological saline by centrifugation, and then centrifuged in deio nized water to be subjected to complete hemolysis. For the 100 samples of completely hemolyzed blood, various test items were measured, and at the same time the degree of hemolysis (the hemoglobin concentration) was measured. The degree of hemolysis was determined by, as in US-A- 4263512, measuring a spectrum for hemoglobin in the visible wavelength region from the difference between light absorbances at two wavelengths of 570 nm and 600 nm, and making a correction for the influence of chyle.

In the step 102, the correction factor based on the degree of hemolysis is recognized as a value with a tolerance. The factor (the slope) in the middle of the tolerance zone and the tolerance are determined for each facility for examination, and each of inpatient group, group medical examination group, infant group, adult group, men group and women group. An error function graph is obtained by plotting the degree of hemolysis along the x axis and the measured concentration of each test item along the y axis.

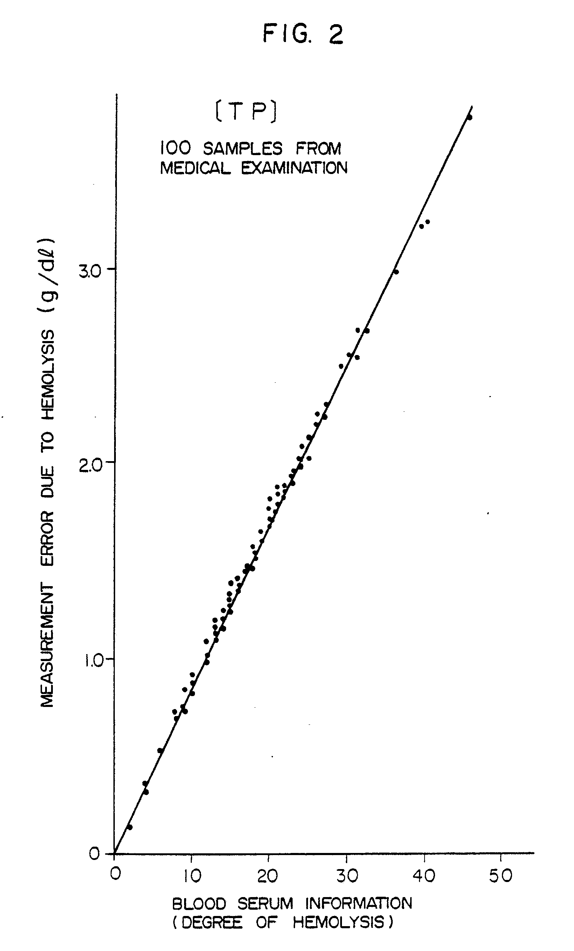

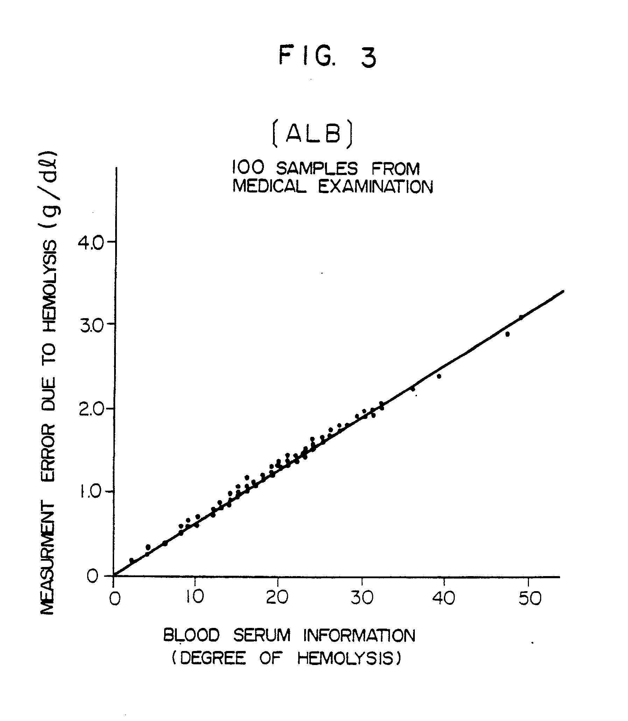

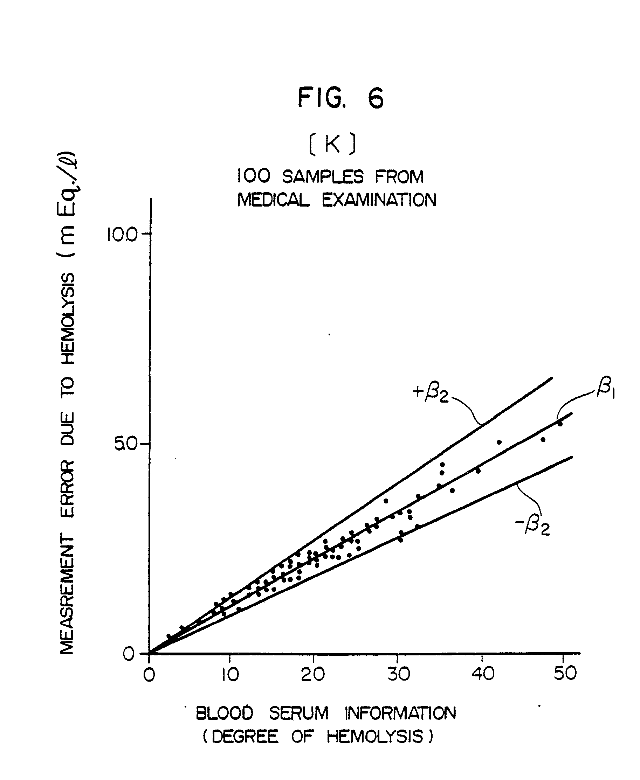

Typical examples of error function graph are shown in Figs. 2 to 6. These figures are, in that order, for total protein (TP), albumin (ALB), LDH, GOT and K, respectively. TB and ALB are typical cases where hemoglobin released by hemolysis acts as an interfering chromogen, and the relationship between the degree of hemolysis and measured values of TP or ALB (which are substantially errors in TP or ALB measurement due to hemolysis) is in the manner of complete distribution on a straight line. Components showing such a relationship include, besides them, total cholesterol, free cholesterol, uric acid, triglycerides, sodium, chloride, - ß-lipoproteins, thymol turbidity test, zinc sulfate turbidity test, phospholipids and free fats (i.e. fats free from other componants and composed of only fat componant. The relationships for these components are all in the manner of distribution on a straight line though different in the slope of the straight line.

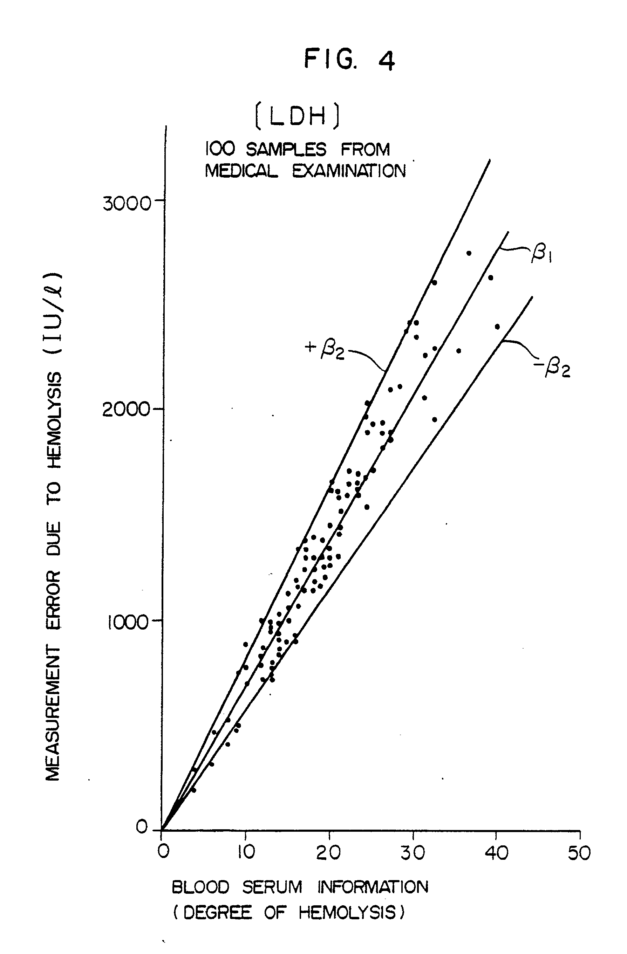

On the other hand, in the case of LDH, GOT and K shown in Figs. 4 to 6, respectively, the measurement errors tends to be increased in proportion to an increase of the degree of hemolysis as in the case of TP and ALB, but the relationship between the degree of hemolysis and measured values of LDH, GOT or K is in the manner of distribution which is not on a straight line but in a considerably wide range. Therefore, the tolerance $(+\beta_2, -\beta_2)$ is determined so as to include all the measured values. The middle of tolerance zone is expressed as the slope $\beta_1$.

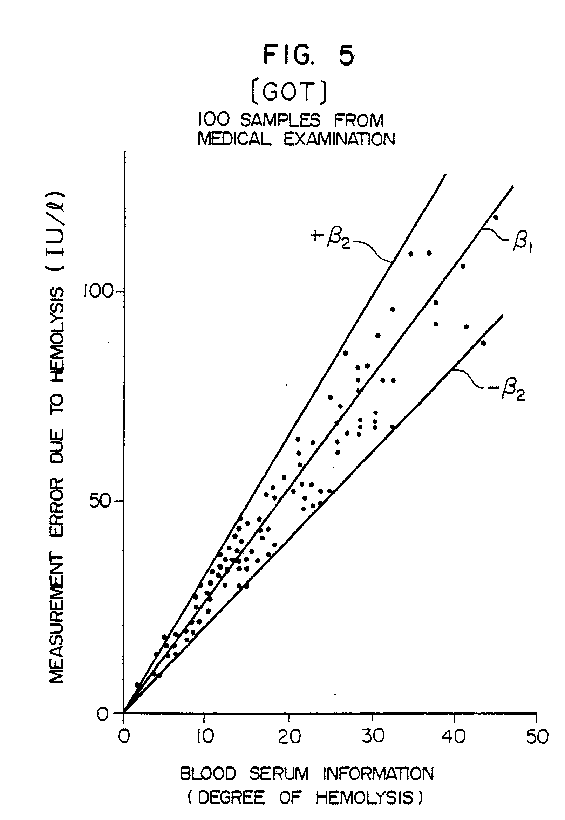

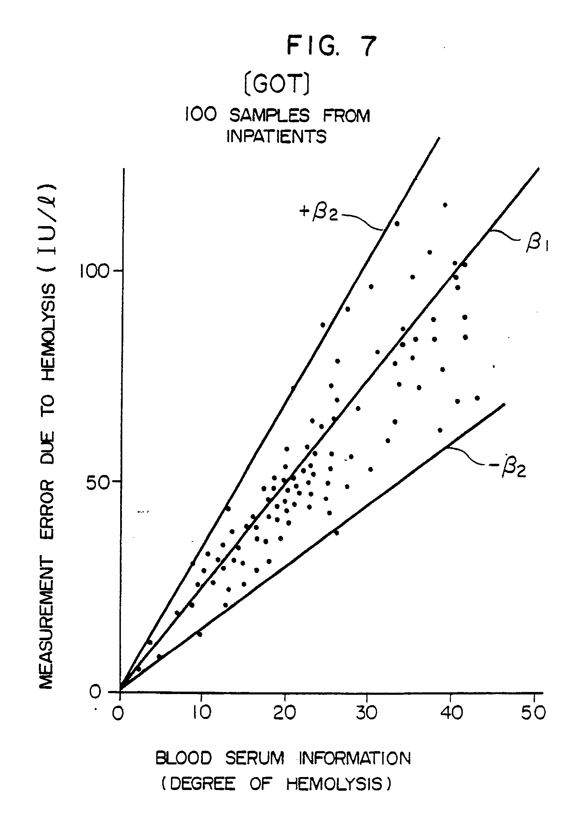

In the step 103, the slope $\beta_1$ of error function and the tolerance $_2$ are determined for each test component. The slope and the tolerance depend on the relationship between the concentrations of the component, i.e., LDH or GOT and hemoglobin in red blood cells and those in blood plasma (blood serum). Therefore, the tolerance $2\beta_2$ and the median of slope $\beta_1$ vary considerably depending on which subjects are used, healthy persons under group medical examination or inpatients. In the case of group medical examination, they vary depending on which subjects are used, schoolchildren or adults (schoolchildren and adults are different from each other by about 10% in the hemoglobin concentration in red blood cells). Similarly, men and women are different from each other by about 12% in the hemoglobin concentration in red blood cells. For example, Fig. 7 shows the results of measurement under the same conditions as in Fig. 5 for 100 inpatients. The slope $\beta1$ in the middle of the positive error range of GOT due to hemolysis in Fig. 7 is substantially the same as in Fig. 5 but the tolerance $2\beta_2$ in Fig. 7 shows a distribution as wide as about 1.7 times that shown in Fig 5 for healthy persons.

That is to say, in order to make the most effective correction, the correction factors $\beta_1$ and $\beta_2$ should be determined by carrying out such experiments as shown in Figs. 2 to 7, for samples obtained from inpatients and outpatients or those obtained in medical examinations on adults and schoolchildren in each facility.

In the step 104, the slope $\beta_1$ and the tolerance $\beta_2$ are input for each component to a data processor from the operating pannel of an automatic biochemical analyzer. Their values are stored in the storage device of a microcomputer. In inputting the measurement conditions, a correction factor for turbidity (chyle) and a correction factor $\gamma$ for bilirubin, e.g., icterus are also input depending on the kind of sample. In this case, $\alpha$ and $\gamma$ are constant for each component. In the case of a component whose concentration is lower in red blood cells than in blood serum, zero is input as $\beta_2$ because it is sufficient that correction is made for the influence of hemoglobin as a disturbing chromogen.

When measuring operations of the analyzer are initiated, required component are measured, as in the step 105, for each sample on the basis of item selection information previously input for each serum sample (including serum samples which have undergone hemolysis). The measured values before correction are stored in the storage device of a microcomputer.

The step 106 is carried out substantially simultaneously with the step 105. In the step 106, such a specific component that the light absorption of an objective substance is in the ultraviolet region is selected, and the absorption in the visible region by a sample solution of the specific component is measured by a suitable three pairs of dual wavelength methods, whereby there are measured blood serum informations, i.e., the degree of turbidity, the degree of hemolysis (the hemoglobin concentration) and the degree of icterus (the bilirubin concentration) of a sample serum. This measuring method is the same as in US-A-4263512.

As a suitable component to be tested for investigating the serum conditions, there is preferably exemplified one selected from components which are such that the absorption of the correspoding objective substance is not in the ultraviolet region and that the frequency of measurement to be required is as high as possible. The sample to be used is preferably the same as that used for measuring a component of a sample to be analyzed, (in order to reduce a reagent loss accompanying the measurement of the component of blood serum sample). Components satisfying these conditions include GOT, GTP, and LDH, the objective substance for which is NADH (maximal absorption: 340 nm). A method for measuring the blood serum components comprises, for example, measuring the conversion rate of NADH by use of two wavelengths (e.g., 340 nm and 376 nm) in the ultraviolet region for GOT sample to be measured to determine the GOT activity, simultaneously measuring the degree of turbidity at two wavelengths (e.g., 660 nm and 700 nm) in the long wavelength region where no absorption due to hemoglobin or bilirubin occurs, determining the degree of hemolysis from the degree of turbidity and absorptions at two wavelengths (570 nm and 600 nm) in the middle wavelength region where no absorption due to bilirubin occurs, and determining the degree of icterus from the degree of turbidity, the degree of hemolysis and absorption at two wavelengths (e.g., 480 nm and 505 nm) in the short wavelength region where absorption due to bilirubin occurs.

4

In step 107, a measured value of each component before correction is corrected using the degree of turbidity X, degree of hemolysis Y, degree of icterus Z and correction factors $\alpha$, $\beta_1$, $\beta_2$ and $\gamma$. When the concentration of an component corresponding to a component in red blood cells is high, namely, when a componant whose tolerance $\beta_2$ is not zero is measured, a corrected value with a tolerance of ($\pm\beta_2 \cdot Y$) is given to this component. Correction calculation is performed according to the equation:

$$C' \pm \Delta C' = C - dX - (\beta_1 \pm \beta_2) Y - \gamma Z$$

wherein C is a measured value of each item before correction, and $C' \pm \Delta C'$ is a value with a tolerance of each component after correction.

In step 108, a correction result ($C' \pm \Delta C'$) with a tolerance is output and displayed for each component. The output value of a component whose input of $\beta_2$ is not zero is reported by data with a significant tolerance. However, when the degree of hemolysis Y is zero, $\Delta C'$ becomes zero, so that an output value without tolerance is reported.

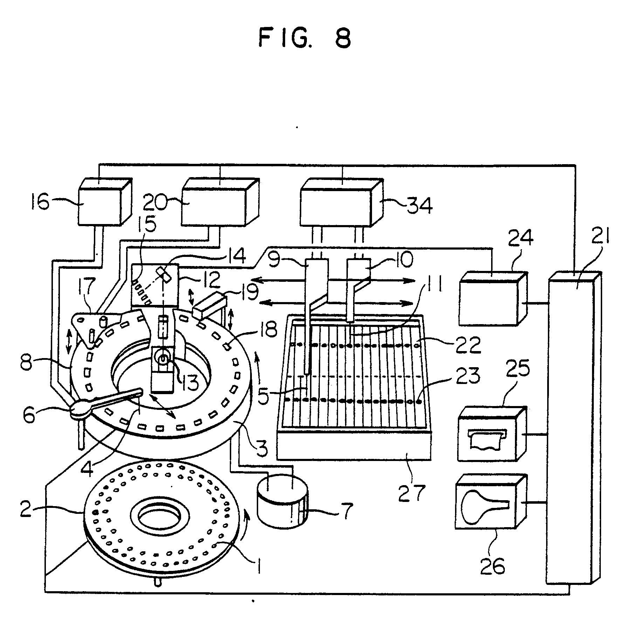

Next, an automatic biochemical analyzer to which this invention has been applied is explained with reference to Fig. 8. This automatic analyzer is a so-called overall-reaction process measurement type analyzer which permits easy input of measurement conditions by the CRT conversational mode, have a multi-wavelength photometer and a turntable-type group both for reaction and for photometry, and permits measurement of absorbance for each vessel at regular intervals.

This analyzer can be equipped with a sample table 2 which holds a predetermined number of sample containers 1 and can be rotated in the correct direction or the reverse direction and stopped at an optional position, and a predetermined number of reaction containers 18 which are transparent and double as photometric cuvettes. Said analyzer has a reaction table 8 which can be rotated in the forward or reverse direction and stopped at an optional position; a sampling mechanism 6 which communicates with a sample syringing mechanism 16 by tubing, aspirates a predetermined amount of a sample solution from a sample container 1 at a predetermined position on the sample table 2, and discharges the same into a reaction container 18 at a predetermined position on the reaction table 8; and a suction-and-discharge nozzle 4 thereof. In addition, said analyzer has a reagent pipetting system which select a required reagent from a first reagent group 22 and a second reagent group 23 set in a refrigerator 27 and pipettes the same into said reaction containers by means of a first-reagent pipetting mechanism 9 and a second-reagent pipetting mechanism 10 both communicating via tubing with a syringe mechanism for pipetting reagents and suction-and-discharge nozzles 5 and 11 thereof, at the time when each reaction container in which said predetermined amount of sample has been collected depending on components proceeds with the operation cycle and stops at a predetermined position; and a stirring mechanism 19 for stirring a solution contained in each reaction container.

A spectroscope 12 comprising a light source 13, a flat diffraction grating 14 and a plurality of light semiconductor detectors 15 is placed so as to insert therein a row of reaction containers on the reaction table 8. Said analyzer has a multi-wavelength photometric system by which the absorbances of reaction solutions in all the reaction containers which go across the optical axis at the time of rotation of the reaction table 8 are measured at two wavelengths previously input for each test item; a photometric-system controller 24 comprising a multiplexer which selects wavelengths, a LOG amplifier which processes signals and, an A/D converter ; a washing mechanism 17 which communicates, by tubing, with a water supply and discharge mechanism 20 for washing and washes each reaction container which has been subjected to measurement, for reuse; a constant temperature water bath 3 which is placed under the reaction table and keeps each reaction container at a constant temperature (usually 37°C); a constant temperature water circulator 7 communicating therewith; a main controller 21 comprising a microcomputer which controls all these mechanisms and systems and performs various data calculation and input-output control; a printer 25; and a CRT screen 26.

Said analyzer is an overall-reaction process photometry type analyzer in which for each operation cycle, the reaction table 8 makes one revolution plus revolution corresponding to one reaction container (referred to as 1 pitch) and stops for a predetermined period of time, and in concert with the rotation and stoppage, there are carried out collection of a sample serum, add ition of a first reagent and a second reagent, stirring, washing, and photometry (only the photometry is conducted during the rotation and the other operations are conducted during the stoppage). The same structure as described above is disclosed in US-A- 4451433 or EP-A-0052006.

Such an automatic analyzer of overall-reaction process photometry type is advantageous for measuring blood serum informations and thereby making the various corrections described above. This is because although in this analyzer, the absorbances for all the reaction containers are measured at regular intervals

5

from immediately after the addition of a first reagent, photometric data actually required for determining the enzymic activities of e.g. GOT, GPT, and LDH. described above are only data obtained after the addition of a second reagent. Therefore, photometry from the addition of a first reagent to that of a second reagent need not be carried out in the ultraviolet region (340 nm and 376 nm) suitable for the objective substance NADH and can be carried out at three pairs of dual wavelengths which are necessary for calculating blood serum informations. Moreover, about 15 times (on a 20-second cycle for 5 minutes) of photometry can be carried out until the addition of a second reagent, and by the above-mentioned three dual wavelength methods, plural times (for example, 5 times) of photometry can be carried out for each method, resulting in precise mesurement of the blood serum informations and hence a precise corrected value of each component.

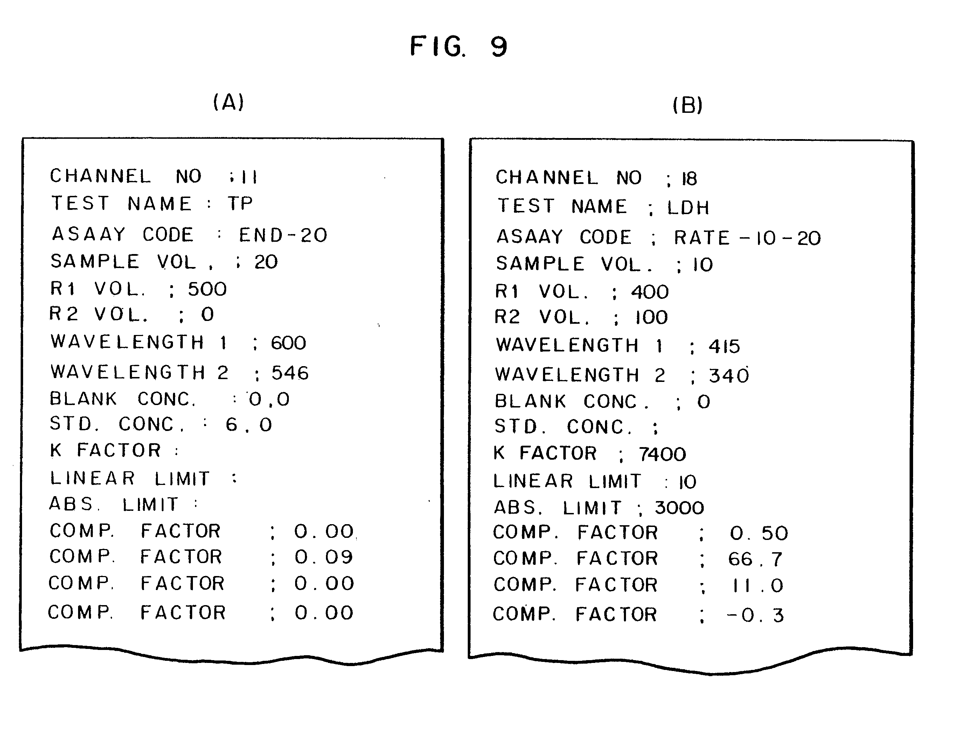

Examples of input of measurement conditions are shown in Fig. 9 (A) and (B) by taking the cases of TP and LDH. That is to say, the present analyzer has a mechanism which permits input of measurement conditions such as an assay method (ASSAY CODE in Fig. 9), the collecting volume of a sample (SAMPLE VOL.), the pipetting volumes of first and second reagents (R1 VOL. and R2 VOL.), measuring wavelengths (WAVELENGTH 1 and WAVELENGTH 2), the concentrations of blank solutions and standard solutions (RGT. BLANK CONC. and STD. CONC.), K factor (FACTOR), limit absorbance for checking insufficiency of substrate (ABS. LIMIT), linearity check tolerance (LINEARITY CHECK), and factors for correction by the above-mentioned blood serum components (COMPENSATE FACTOR $\alpha$, ditto $\beta_1$, ditto $\beta_2$ and ditto $\gamma$).

Examples of input of the factors $\alpha$ to $\gamma$ for typical components are shown in Table 1. The factors $\beta_1$ and $\beta_2$ were experimentally determined for healthy persons (irrespective of sex and age) under a medical examination.

6

## Table 1 Examples of input of correction factors

| Test item / Component | Correction factor | | | |
|---|---|---|---|---|
| | $\alpha$ | $\beta_1$ | $\beta_2$ | $\gamma$ |
| LDH | 0.5 | 66.7 | 11.0 | −0.3 |
| K | 0.0 | 0.12 | 0.02 | 0.0 |
| GOT | 0.0 | 2.5 | 0.6 | 0.0 |
| ZTT | 0.02 | 0.21 | 0.00 | 0.06 |
| TTT | 0.30 | 0.18 | 0.00 | 0.01 |
| TG | 0.0 | 2.5 | 0.0 | 0.0 |
| $\gamma$-GTP | 0.0 | 0.7 | 0.0 | −0.1 |
| GPT | 0.0 | 0.3 | 0.1 | 0.0 |
| UA | 0.01 | 0.08 | 0.00 | 0.00 |
| $\beta$-L | 6.7 | 7.7 | 0.0 | 0.7 |
| ALB | 0.00 | 0.06 | 0.00 | −0.01 |
| CHO | 0.0 | 0.4 | 0.0 | −0.2 |
| TP | 0.00 | 0.09 | 0.00 | 0.00 |
| IP | 0.00 | 0.10 | 0.00 | −0.01 |

In Table 2 are shown the results of actual measurement by means of the apparatus shown in Fig. 8 to which the correction factors shown in Table 1 has been input. As samples, blood sera of hemolyzed blood were selected among blood sera for medical examination. As measured values, both uncorrected values (conventional measured values) and corrected values are shown together with measured values of blood serum components.

EP 0 268 025 B1

Table 2  Comparison between measured values of a
conventional method and those by the present method

| No. | Measured values of blood serum information | | | Measured values of typical *components* (items): upper row (conventional method), lower row (the present method) | | | | |
|---|---|---|---|---|---|---|---|---|
| | Degree of chyle | Degree of hemolysis | Degree of icterus | LDH | K | GOT | TP | ALB |
| 1 | 0 | 11 | 4 | 1150<br>416 ± 121 | 5.2<br>3.9 ± 0.2 | 45<br>13 ± 7 | 7.8<br>6.8 | 4.3<br>3.6 |
| 2 | 0 | 5 | 2 | 720<br>386 ± 55 | 5.1<br>4.5 ± 0.1 | 38<br>21 ± 4 | 7.5<br>7.2 | 4.6<br>4.3 |
| 3 | 1 | 20 | 7 | 1573<br>239 ± 220 | 6.7<br>4.3 ± 0.4 | 77<br>27 ± 12 | 9.6<br>7.8 | 5.5<br>4.3 |
| 4 | 2 | 7 | 3 | 794<br>327 ± 77 | 5.5<br>4.6 ± 0.1 | 47<br>29 ± 4 | 8.1<br>7.5 | 4.7<br>4.3 |
| 5 | 0 | 3 | 6 | 541<br>341 ± 33 | 4.6<br>4.2 ± 0.1 | 42<br>34 ± 2 | 6.9<br>6.6 | 4.8<br>4.6 |
| 6 | 1 | 6 | 5 | 679<br>279 ± 66 | 4.9<br>4.1 ± 0.1 | 21<br>6 ± 4 | 7.9<br>7.4 | 5.1<br>4.7 |
| 7 | 1 | 15 | 4 | 1246<br>245 ± 165 | 6.2<br>4.4 ± 0.3 | 81<br>43 ± 9 | 9.1<br>7.7 | 5.7<br>4.8 |
| 8 | 0 | 6 | 16 | 618<br>218 ± 66 | 4.6<br>3.9 ± 0.1 | 55<br>40 ± 4 | 7.5<br>7.0 | 3.9<br>3.5 |

- Cont'd -

Table 2 (Cont'd)

| 9 | 0 | 8 | 3 | 905<br>371 ± 88 | 5.1<br>4.1 ± 0.2 | 58<br>38 ± 5 | 7.6<br>6.9 | 4.3<br>3.8 |
|---|---|---|---|---|---|---|---|---|
| 10 | 3 | 32 | 2 | 2216<br>79 ± 352 | 7.7<br>3.9 ± 0.6 | 114<br>34 ± 19 | 10.3<br>7.4 | 5.6<br>3.7 |

EP 0 268 025 B1

As shown in Table 2, in the case of a conventional method, all the samples showed abnormally high value of LDH much higher than normal value (130 -520 IU/liter) because of release of enzymes from red blood cells. Furthermore, the samples including a high degree of hemolysis such as No. 1, No. 3, No. 7 and No. 10 show very abnormal values of more than 1000 IU/liter. Such measured values are utterly meaningless for clinical diagnosis, and what is worse, when they are reported as such, there is a very high possibility of their causing a seriously erroneous diagnosis. On the other hand, the measured values by this invention have a specific tolerance proportional to the degree of hemolysis but clearly indicate that the LDH activities of all the samples are within or near the normal range. Since all the samples were obtained from healthy persons (a medical examination), concentration of all the values after correction according to this invention in or near the normal range indicates the reasonability of these values, and these values can be said to be sufficiently significant measured values for diagnosis.

Substantially the same results as described above are shown also for K (normal value: 3.7 - 4.8 m mol/liter) and GOT (normal value: 7 - 38 IU/liter).

That is to say, it can be seen that the present example is a very effective method for items whose concentrations in blood cells are high, such as LDH, K and,GOT.

TP and ALB are typical examples of components to which a conventional two-point assay method cannot be applied because hemoglobin released by hemolysis acts as a interfering chromogen simply but hemoglobin and turbid particles are denatured or decomposed owing to the solubility of measuring reagents (these reagents contain a high concentration of a surface active agent). The results shown in Table 2 indicate that the correction by blood serum informations according to this invention is effective also for such components.

Components which are most important in biochemical examination but whose measured value for a hemolyzed sample has been utterly inapplicable to diagnosis because of their high concentrations in red blood cells, such as LDH, HBDH, GOT, GPT and, K could be improved so as to be usable for diagnosis by correcting them by the method according to this invention.

This improvement is very effective when there is considered the present situation of laboratories in which the percentage occurrence of samples which have undergone medium or higher degree of hemolysis is as high as several per cent to 10 per cent even when the greatest care is taken in collecting blood. The improvement is not only advantageous to clinical medicine and hospitals including laboratories but also very effective in reducing physical pain inflicted on patients by recollection of blood due to hemolysis.

## Claims

1. A method for analyzing a blood sample by the correction of measured values of analytes for the degree of hemolysis, wherein the degree of hemolysis for said blood sample to be analyzed is measured and said correction is
**characterized** by the steps of
   - obtaining an uncorrected measured value of a component of a blood serum or plasma sample to be analyzed; and
   - correcting said uncorrected measured value by the measured degree of hemolysis for said blood serum or plasma sample on the basis of the previosly-established correlation between the degree of hemolysis and the amount of said componant which is released by destruction of red blood cells and which is the same as said componant to be determined in the blood serum or plasma sample to be analyzed.

2. The method for analyzing a blood sample according to Claim 1, wherein said correlation is previously stored in a computer.

3. The method for analyzing a blood sample according to Claim 2, wherein the value of said error is calculated by said computer on the basis of said correlation stored therein.

4. The method for analyzing a blood sample according to Claim 1, wherein the measured value of said componant which has been corrected by the value of said error is output in a display device.

5. The method for analyzing a blood sample according to Claim 1, wherein said corrected value is displayed as a value with a tolerance.

6. The method for analyzing a blood sample according to Claim 1, wherein said correlation is established on the basis of data obtained for a group of members similar in health condition, a group of members similar in age, or a group of members of the same sex.

7. The method for analyzing a blood sample according to Claim 1 or 6, wherein said correlation is established from the results of measurement obtained for a large number of actual samples.

8. Th e method for analyzing a blood sample according to Claim 6, which is carried out by storing a plurality of said correlations for said individual groups in a computer, and selecting a group to be applied, from said groups depending on said sample to be analyzed.

9. The method for analyzing a blood sample according to Claim 1, wherein said component is selected from the group consisting of total protein, albumin, lactate dehydrogenase (LDH), GOT, GPT, potassium, total cholesterol, free cholesterol, uric acid, triglycerides, sodium, chloride, ß-lipoproteins, thymol turbidity test, zinc sulfate turbidity test, phospho lipid, and free fats.

10. The method for analyzing a blood sample according to Claim 1, wherein said component is one whose concentration is higher in red blood cells than in blood serum or blood plasma.


**Revendications**

1. Procédé pour analyser un échantillon sanguin moyennant la correction des valeurs mesurées des analytes en fonction du degré d'hémolyse, et selon lequel on mesure le degré d'hémolyse pour ledit échantillon sanguin devant être analysé et ladite correction étant
caractérisée par les étapes consistant à
    - obtenir une valeur mesurée non corrigée d'un constituant d'un échantillon de sérum sanguin ou de plasma devant être analysé; et
    - corriger ladite valeur mesurée non corrigée au moyen du degré mesuré d'hémolyse pour ledit échantillon de sérum sanguin ou de plasma sanguin sur la base de la corrélation, préalablement établie, entre le degré d'hémolyse et la quantité dudit constituant qui est libéré par la destruction de globules rouges et est identique au constituant devant être déterminé dans l'échantillon de sérum sanguin ou de plasma sanguin devant être analysé.

2. Procédé pour analyser un échantillon sanguin selon la revendication 1, selon lequel on mémorise ladite corrélation préablement dans un ordinateur.

3. Procédé pour analyser un échantillon sanguin selon la revendication 2, selon lequel ledit ordinateur calcule la valeur de ladite erreur sur la base de ladite corrélation mémorisée en lui.

4. Procédé pour analyser un échantillon sanguin selon la revendication 1, selon lequel la valeur mesurée dudit constituant, qui a été corrigée de la valeur de ladite erreur, est délivrée dans un dispositif d'affichage.

5. Procédé pour analyser un échantillon sanguin selon la revendication 1, selon lequel ladite valeur corrigée est affichée en tant que valeur affectée d'une tolérance.

6. procédé pour analyser un échantillon sanguin selon la revendication 1, selon lequel ladite corrélation est établie sur la base des données obtenues pour un groupe de patients ayant des conditions de santé similaires, un groupe de patients ayant des âges similaires ou un groupe de patients du même sexe.

7. Procédé pour analyser un échantillon sanguin selon la revendication 1 ou 6, selon lequel ladite corrélation est établie à partir des résultats de mesure obtenus pour un nombre élevé d'échantillons réels.

8. Procédé pour analyser un échantillon sanguin selon la revendication 6, qui est mis en oeuvre au moyen de la mémorisation d'une pluralité desdites corrélations pour lesdits groupes individuels dans un ordinateur et au moyen de la sélection d'un groupe devant être utilisé, parmi lesdits groupes, en fonction dudit échantillon devant être analysé.

9. Procédé pour analyser un échantillon sanguin selon la revendication 1, selon lequel on choisit ledit constituant dans le groupe incluant la protéine totale, l'albumine, la lactate-déshydrogénase (LDH), la GOT, la GPT, le potassium, le cholestérol total, le cholestérol libre, l'acide urique, les triglycérides, le sodium, le chlore, les ß-lipoprotéines, le test de turbidité au thymol, le test de turbidité au sulfate de zinc, les phospholipides et les graisses libres.

10. Procédé pour analyser un échantillon sanguin selon la revendication 1, dans lequel ledit constituant est un constituant dont la concentration est plus élevée dans les globules rouges que dans le sérum sanguin ou le plasma sanguin.

## Ansprüche

1. Verfahren zum Analysieren einer Blutprobe durch die Korrektur gemessener Probenwerte für den Grad einer Hämolyse, wobei der Grad der Hämolyse für die zu analysierende Blutprobe gemessen wird, und die Korrektur
   **gekennzeichnet ist** durch die Schritte

   - Erhalten eines unkorrigierten gemessenen Wertes einer Komponente eines Blutserums oder einer Plasmaprobe, die zu analysieren sind; und
   - Korrigieren des unkorrigierten gemessenen Wertes durch den gemessenen Grad der Hämolyse fär das Blutserum oder die Plasmaprobe auf der Basis der zuvor gebildeten Korrelation zwischen dem Grad der Hämolyse und dem Betrag der Komponente, die durch eine Zerstörung der roten Blutzellen freigesetzt ist, und die die gleiche wie die Komponente ist, die in dem Blutserum oder der Probe zu bestimmen ist, die zu analysieren sind.

2. Verfahren zum Analysieren einer Blutprobe nach Anspruch 1, wobei die Korrelation vorher in einem Computer gespeichert wird.

3. Verfahren zum Analysieren einer Blutprobe nach Anspruch 2, wobei der Wert des Fehlers durch den Computer auf der Basis der darin gespeicherten Korrelation berechnet wird.

4. Verfahren zum Analysieren einer Blutprobe nach Anspruch 1, wobei der gemessene Wert der Komponente, die durch den Wert des Fehlers korrigiert worden ist, in einer Anzeigevorrichtung ausgegeben wird.

5. Verfahren zum Analysieren einer Blutprobe nach Anspruch 1, wobei der korrigierte Wert als ein Wert mit einer Toleranz angezeigt wird.

6. Verfahren zum Analysieren einer Blutprobe nach Anspruch 1, wobei die Korrelation auf der Basis von Daten gebildet wird, die für eine Gruppe von Zugehörigen erhalten werden, die gleich im Gesundheitszustand sind, für eine Gruppe von Zugehörigen, die gleich im Alter sind, oder eine Gruppe von Zugehörigen desselben Geschlechts.

7. Verfahren zum Analysieren einer Blutprobe nach Anspruch 1 oder 6, wobei die Korrelation aus den Ergebnissen der Messung gebildet wird, die für eine große Anzahl von aktuellen Proben erhalten wird.

8. Verfahren zum Analysieren einer Blutprobe nach Anspruch 6, das durch Speichern einer Vielzahl der Korrelationen für die individuellen Gruppen in einem Computer und Auswählen einer Gruppe aus den Gruppen, auf die es angewandt werden soll, in Abhängigkeit von der zu analysierenden Probe, ausgeführt wird.

9. Verfahren zum Analysieren einer Blutprobe nach Anspruch 1, wobei die Komponente aus der Gruppe

12

ausgewählt wird, die aus ganzem Protein, Albumin, Laktatdehydrogenase (LDH), GOT, GPT, Kalium, ganzem Cholesterin, freiem Cholesterin, Harnsäure, Triglyzeriden, Natrium, Chlorid, ß-Lipoproteine, Thymoltrübheitstest, Zinksulfattrübheitstest, Phospholipid und freien Fetten besteht.

10. Verfahren zum Analysieren einer Blutprobe nach Anspruch 1, wobei die Komponente eine ist, deren Konzentration in roten Blutzellen höher ist, als in Blutserum oder Blutplasma.

# FIG. 1

MEASUREMENT FOR HEMOLYZED BLOOD OF MANY SUBJECTS (THE DEGREE OF HEMOLYSIS AND EACH TEST ITEM) — 101

PREPARATION OF AN ERROR FUNCTION GRAPH  $X$ AXIS... THE DEGREE OF HEMOLYSIS  $Y$ AXIS... MEASURED VALUE OF EACH ITEM — 102

DETERMINATION OF SLOPE $\beta_1$ AND TOLERANCE $\beta_2$ OF ERROR FUNCTION FOR EACH ITEM — 103

INPUT OF MEASUREMENT CONDITIONS INCLUDING $\beta_1$ AND $\beta_2$ FOR EACH ITEM — 104

AUTOMATIC MEASUREMENT OF EACH ITEM ON THE BASIS OF ITEM SELECTION INFORMATION, FOR EACH SAMPLE — 105

ANALYSIS OF SPECTRA OF GOT, GPT, ETC. IN THE VISIBLE REGION, AND AUTOMATIC MEASUREMENT OF THE DEGREE OF HEMOLYSIS OF EACH SAMPLE — 106

CALCULATION FOR CORRECTION OF EACH ITEM BY THE DEGREE OF HEMOLYSIS
$$C' \pm \Delta C' = C - \alpha X - (\beta_1 \pm \beta_2) Y - \gamma Z$$ — 107

OUTPUT OF A CORRECTED VALUE WITH A TORERANCE ( $C' \pm \Delta C'$ ) FOR EACH ITEM — 108

FIG. 2

# F I G. 3

(ALB)

100 SAMPLES FROM
MEDICAL EXAMINATION

MEASURMENT ERROR DUE TO HEMOLYSIS (g/dℓ)

BLOOD SERUM INFORMATION
(DEGREE OF HEMOLYSIS)

# FIG. 4

(LDH)

100 SAMPLES FROM MEDICAL EXAMINATION

MEASUREMENT ERROR DUE TO HEMOLYSIS (IU/ℓ)

BLOOD SERUM INFORMATION
(DEGREE OF HEMOLYSIS)

FIG. 5

[GOT]

100 SAMPLES FROM
MEDICAL EXAMINATION

FIG. 6

[K]

100 SAMPLES FROM
MEDICAL EXAMINATION

FIG. 7

[GOT]

IOO SAMPLES FROM
INPATIENTS

FIG. 8

# FIG. 9

### (A)

```
CHANNEL  NO  ;11
TEST  NAME : TP
ASAAY  CODE  : END-20
SAMPLE  VOL ,  ; 20
R1  VOL.  ; 500
R2  VOL.  ; 0
WAVELENGTH 1  ; 600
WAVELENGTH 2  ; 546
BLANK  CONC.    : 0 ,0
STD.  CONC.  : 6 . 0
K  FACTOR :
LINEAR  LIMIT  :
ABS.  LIMIT :
COMP.  FACTOR    ; 0. 00
COMP.  FACTOR    ; 0. 09
COMP.  FACTOR    ; 0. 00
COMP.  FACTOR    ; 0. 00
```

### (B)

```
CHANNEL  NO  ; 18
TEST  NAME  ; LDH
ASAAY  CODE  ; RATE - 10 - 20
SAMPLE  VOL.  ; 10
R1  VOL.  ; 400
R2  VOL.  ; 100
WAVELENGTH 1  ; 415
WAVELENGTH 2  ; 340
BLANK  CONC.  ; 0
STD.  CONC.  ;
K  FACTOR  ; 7400
LINEAR  LIMIT  : 10
ABS.  LIMIT ; 3000
COMP.  FACTOR    ; 0. 50
COMP.  FACTOR    ; 66 . 7
COMP.  FACTOR    ; 11 . 0
COMP.  FACTOR    ; -0 . 3
```

EP 0 268 025 B1